# EUROPEAN PATENT APPLICATION

(11) **EP 2 174 601 A1**
(43) Date of publication of application: **14.04.2010**
(21) Application number: 09172568.9
(22) Date of filing: 08.10.2009
(51) Int. Cl.: A61B 17/32, A61B 17/24

(54) **A laryngeal microsurgery operating instrument**

(30) Priority: 09.10.2008 IT MI20081783
(71) Applicant: Guglielmetti, Ruggero, 28100 Novara (IT)
(72) Inventor: Guglielmetti, Ruggero, 28100 Novara (IT)
(74) Representative: Cicogna, Franco

(57) **Abstract**

A laryngeal microsurgery operating instrument comprises a harmonic generator, as smart-chip technology handle, including a piezoelectric ceramics transducer clamped between two steel cylinders and a plurality of blades with a reduced length and size stem, to fit the instrument to the intended specific surgical application.

## Description

### BACKGROUND OF THE INVENTION

The present invention relates to an operating instrument, which has been specifically designed to be used for performing laryngeal microsurgery operations.

As is known, at present, direct microlaryngoscopy phonosurgery, or direct laryngoscopy endoscopical operations are performed either by cold instruments or laser methods (CO2 - diodes) to recover the patient proper vocal vibrating function.

### SUMMARY OF THE INVENTION

The aim of the present invention is to provide such an improved operating instrument, for performing laryngeal microsurgery operations, which is so designed and sized as to be used both in conventional and in endoscopic surgery operations.

Within the scope of the above mentioned aim, a main object of the invention is to provide such an operating instrument which is adapted to perform the same surgical procedures performed by prior instruments, but with much improved operating results.

Yet another object of the present invention is to provide such an operating instrument allowing to recover the patient vibrating mucous wave in a much less time than prior analogous instruments.

Yet another object of the present invention is to provide such an operating instrument which, owing to its specifically designed features, is very reliable and safe in operation.

According to one aspect of the present invention, the above mentioned aim and objects, as well as yet other objects, which will become more apparent hereinafter, are achieved by an operating surgical instrument, particularly for performing microsurgical laryngeal operations, characterized in that said instrument comprises a harmonic wave generator, a handle including a piezoelectric transducer, and a plurality of blades with a small length and size stem, so as to fit said instrument to target different surgical applications.

A mechanical motion is transferred from the handle to the stem the length of which can be changed depending on the target surgical operation.

The subject system is very flexible from an operating standpoint, since it may be used both in conventional and in endoscopic surgical operations.

In fact, the inventive instrument or apparatus allows, by a simple movement of an ultrasonic frequency blade, to provide a cutting, coagulating and dissecting operation with a minimum thermal loss and carbonization of the tissues, without any risks of damaging tissues adjoining the tissue operated upon.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:
Figure 1 is a side elevation view of a surgical complex or combined operating instrument including a harmonic wave instrument, a suction instrument and an optic fiber displaying device;
Figure 2 is a top plan view of the surgical instrument of figure 1;
Figure 3 is a side elevation view of the operating instrument;
Figure 4 is a front view of the subject operating instrument;
Figure 5 is a rear view of the subject operating instrument;
Figure 6 is a side view of the subject surgical operating instrument with a cutting blade on a top side thereof;
Figure 7 is a detail view showing a detail of the tip, as seen from the top, of the subject complex operating instrument;
   and

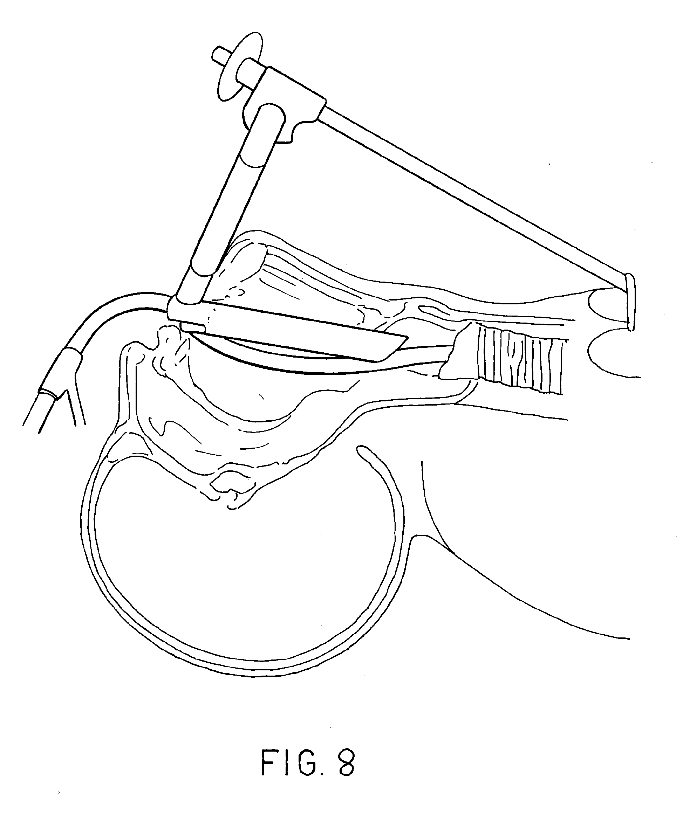

Figure 8 schematically shows the inventive combined surgical instrument in an operation condition thereof.

### DESCRIPTION OF THE PREFERRED EMBODIMENTS

With reference to the number references of the above mentioned figures, the combined surgical operating instrument according to the present invention comprises a harmonic wave instrument, generally indicated by the reference number 1, which can be used jointly with an optical fiber display 2 and a suction instrument 3.

An insulating handle 4 operates as a support for the three above mentioned individual instruments and for the connection 5 of the instrument 1 to the harmonic wave generator or source, not shown in the figures.

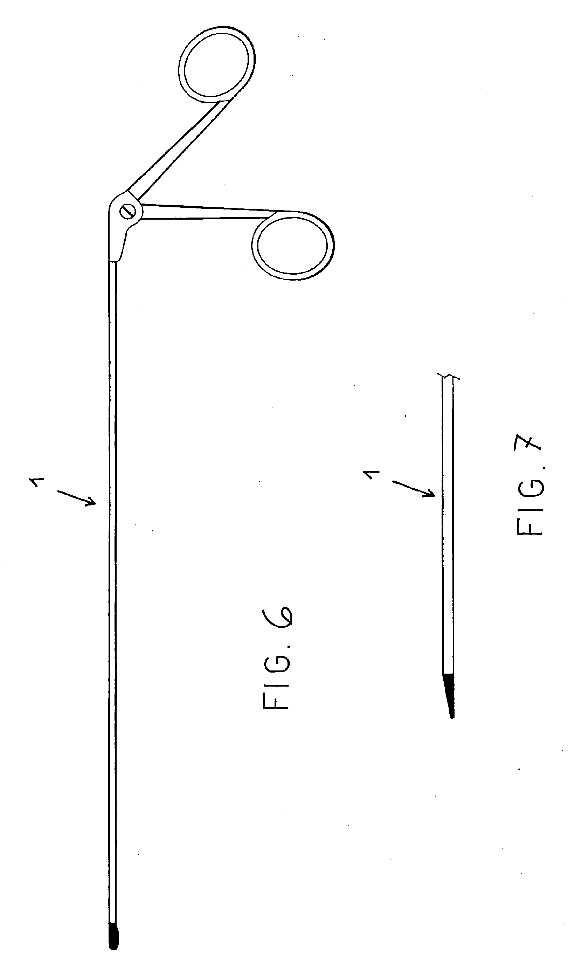

The harmonic wave instrument 1 may comprise, for example, gripper means with a harmonic scalpel for performing microsurgical laryngeal operations in a microlaryngealscopic arrangement, as shown in figures 6 and 7.

The harmonic wave instrument can also comprise a harmonic scalpel vibrating blade for performing microsurgical laryngeal operations according to a microlaryngealscopic arrangement.

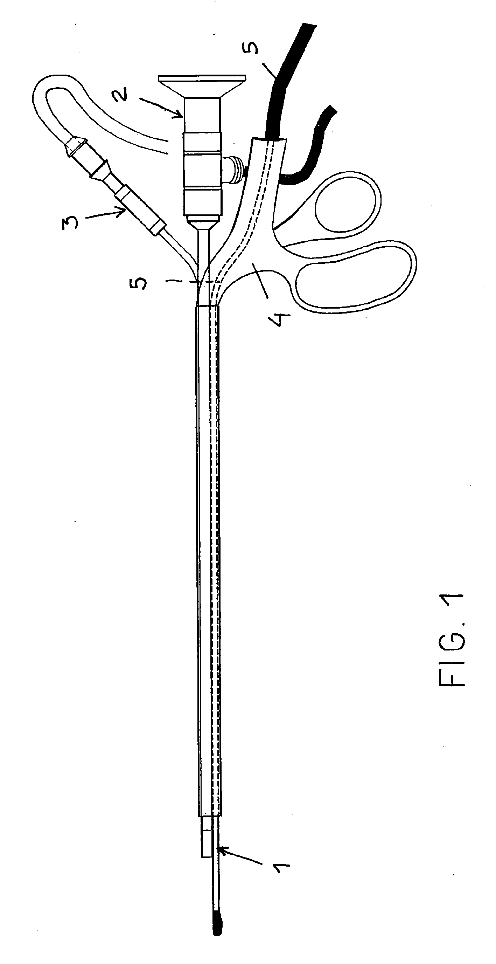

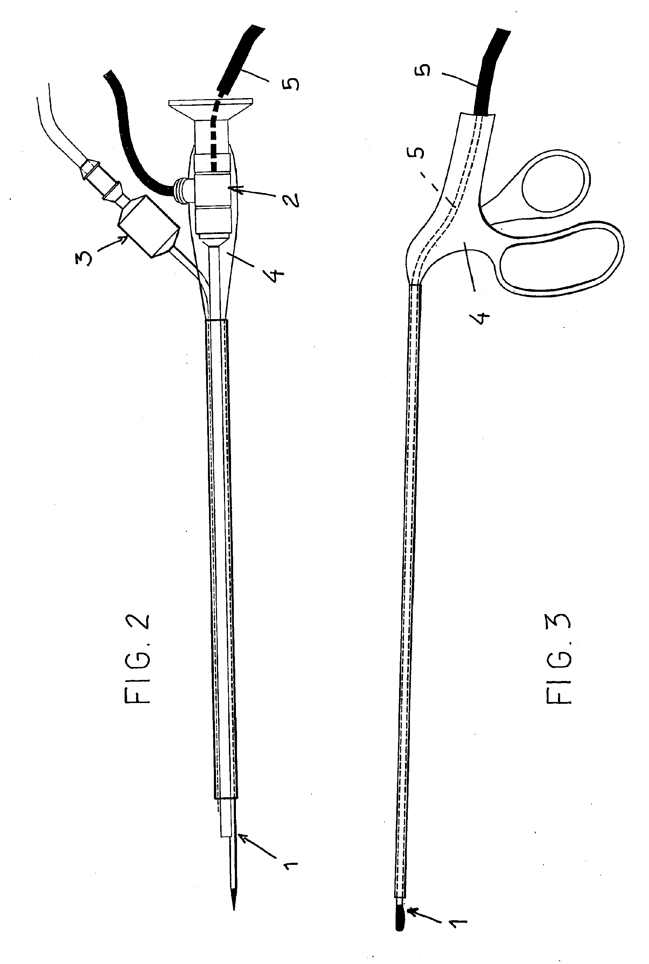

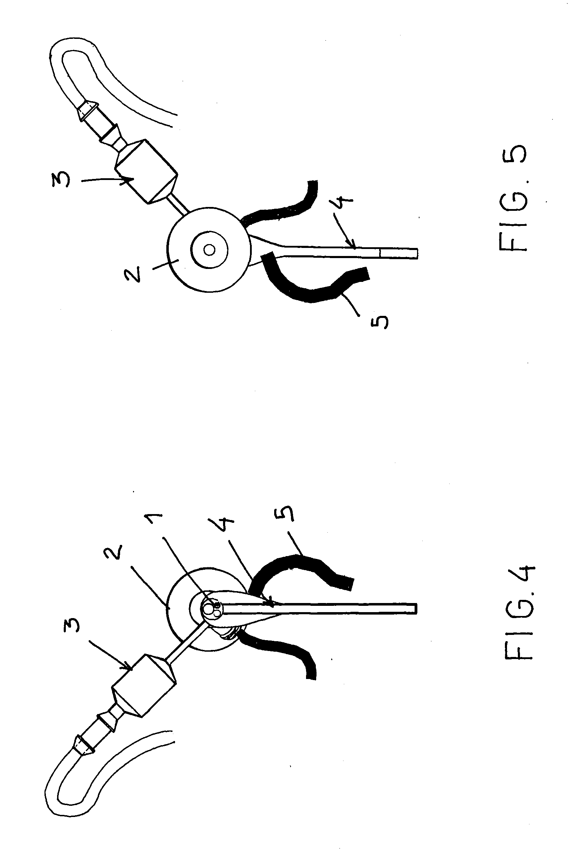

Another embodiment of the harmonic wave instrument, shown in figures 1-5, comprises a blunted hook element, associated with the optic fiber 2 and suction instrument or channel 3, for performing microsurgical laryngeal operations according to a direct video laryngealscopic arrangement.

As is known the harmonic scalpel technology consists of a sound mechanical propagation, in the form of pressure ultrasonic waves, from an energy source to an active blade.

The ultrasonic waves are achieved by transferring electromagnetic energy or power to a piezoelectric transducer which has a physical property of generating mechanical vibrations in response to electric stimulations or magnetic fields.

Low power level ultrasonic waves do not have any effect on tissues and are used for diagnostic purposes (Ultrasonic Imaging).

At large power levels, however, said ultrasonic waves are adapted to produce surgical incisions, haemostasis and dissection.

The cutting haemostasis and dissection system operates at a stable harmonic frequency (for example 55.5 kHz / 55,500 cycles per second) with a maximum blade displacement for example of 50 - 120 µm.

The instrument comprises a generator, a handle including a piezoelectric ceramics transducer clamped between two steel cylinders and a plurality of cutting blades with a stem having a variable length depending on a target surgical application.

A mechanical motion is transferred by the handle to the stem, the length of which changes, as stated, depending on the target surgical operation; this system is very flexible from an operating standpoint since it may be used both in conventional and endoscopic surgery operations.

The above apparatus, in actual practice, allows, by a single movement of an ultrasonic frequency blade, to provide cutting, coagulating and dissecting operations with a minimum thermal dispersion or loss and a minimum tissue carbonization, without any risks of damaging tissues or structures adjacent to those being operated upon.

This effect is an exclusively mechanical effect and the apparatus, while being supplied in electric current, does not provide any electricity flows through the patient and operating personnel, thereby eliminating burning risks due to leakage currents.

Accordingly, it is not necessary to use the conventional electroscalpel plate, with a self evident reduction of the patient and personnel risks, while allowing to save materials and omitting to train operating personnel.

A minimum thermal dispersion or loss means moreover that it is possible to make very accurate cutting and coagulating operations with a maximum operator control, thereby the instrument can also be used near body vessels and structures, without any risks of damaging the latter.

In particular, the effects on the tissues can be of three types, that is a cutting, a coagulating and a dissecting effect.

The principle on which the ultrasonic coagulation is based is similar to that of electrosurgery or laser operations.

The vessels are pressed and closed by a denaturated proteic coagulum, without overheating the tissues with electric and/or light energy.

The protein denaturation effect is achieved by transferring to the tissues a mechanical energy sufficient to break tertiary hydrogen bonds, at about 63°, and accordingly at a temperature less than a temperature of 100°C thereat a destruction of the cells would occur.

The coagulation action is a progressive one, and a secondary heat generation, caused by a molecular inner friction, allows to perform the cutting with a tissue damage which is at least four times less than that caused by an electrosurgical operation, without necrotizing tissues or causing eschars, while favoring a quicker recovery of the tissues.

As the temperature decreases, the coagulum is consolidated, thereby occluding the vessel lumen (coaptive coagulation).

The cutting action is performed in the direction of the pressure applied on the cutting blade; in fact, the vibrating end of the cutting blade will generate a quick variation evaporating intracellular water at a low temperature, a consequent breaking of the cells and, accordingly, a very accurate cutting and dissecting.

Moreover, the steam expanding between the tissue planes (by a cavitation effect) facilitates the tissue separation and a precise identification of the avascular planes to thus perform a bloodless dissection.

It has been found that the invention fully achieves the intended aim and objects.

In fact, it has been found that the inventive surgical harmonic scalpel is adapted to perform the same procedures as those performed by conventional scalpels, but with very improved results.

From fiber optic controls, it has been found that the operated place is devoid of cicatricial outcomes, with a recovery of the vibratile mucous wave much quicker than that of a conventional treatment.

This will allow a correspondingly quicker voice recovery, with a suitable logopedical support, with a vocal result like that of a cold instrument treatment.

The subject harmonic scalpel is adapted to overcome the CO2 laser limits prior laser, which, as is known, is not able of coagulating large size vessel, and the drawbacks of diode lasers operating at high temperatures and with a small cutting precision, thereby the subject harmonic scalpel provides a broad range of operating possibilities for laryngeal pathologies to be endoscopically controlled, while obviating the need of performing outer cuts.

In practicing the invention, the used materials, as well as the contingent size and shapes, can vary, according to requirements.

## Claims

1. An operating surgical combined instrument for performing microsurgical laryngeal operations, **characterized in that** said instrument comprises a harmonic wave generator, a handle including a piezoelectric transducer clamped between two steel cylinders, and a plurality of blades with a variable length and size stem.

2. An operating surgical instrument, according to claim 1, **characterized in that** said operating surgical instrument comprises an optical fiber display and a suction instrument.

3. An operating surgical instrument, according to claim 1, **characterized in that** said handle is an insulating handle operating as a support for a plurality of operating instruments and for coupling said operating instrument to the harmonic wave generator.

4. An operating surgical instrument, according to claim 1, **characterized in that** said instrument comprises a gripper element and a harmonic scalpel for performing microsurgical laryngeal operations according to a microlaryngealscopic arrangement.

5. An operating surgical instrument, according to claim 1, **characterized in that** said instrument comprises a vibrating blade with a harmonic scalpel for performing microsurgical laryngeal operations according to a microlaryngealscopic arrangement.

6. An operating surgical instrument, according to claim 1, **characterized in that** said instrument comprises a blunted hook element, associated with a fiber optic assembly and a suction channel, for performing microsurgical laryngeal operations according to a direct video laryngealscopic arrangement.
